# EUROPEAN PATENT APPLICATION

(11) **EP 4 035 580 A1**
(43) Date of publication of application: **03.08.2022**
(21) Application number: 20869987.6
(22) Date of filing: 18.09.2020
(51) Int. Cl.: A47K 13/24, E03D 9/00

(54) **TOILET SEAT DEVICE**

(30) Priority: 24.09.2019 JP 2019173282; 11.05.2020 JP 2020083273
(71) Applicant: LIXIL Corporation, Koto-ku Tokyo 136-8535 (JP)
(72) Inventor: SHIMAZU, Toshiaki, Tokyo 136-8535 (JP); UEDA, Emi, Tokyo 136-8535 (JP); TANAKA, Kenta, Tokyo 136-8535 (JP); TAKI, Nobuhiro, Tokyo 136-8535 (JP); NISHIGAKI, Hiroshi, Tokyo 136-8535 (JP); NAGATA, Masaaki, Tokyo 136-8535 (JP)
(74) Representative: karo IP
(86) International application number: PCT/JP2020/035580
(87) International publication number: WO 2021/060208

(57) **Abstract**

A toilet device includes a toilet seat (12) disposed on an upper side of a toilet (10); an image sensor (20) provided on a back surface (12a) of the toilet seat and configured to image an inner space of a toilet bowl (11) at time of excretion; an open-close acquiring unit (21) configured to acquire open-close information of the toilet seat; and an imaging control unit configured to determine whether or not to image by the image sensor according to the open-close information of the toilet seat acquired by the open-close acquiring unit.

## Description

### [TECHNICAL FIELD]

The present disclosure relates to a toilet seat device. Priority is claimed on Japanese Patent Application No. 2019-173282, filed September 24, 2019 and Japanese Patent Application No. 2020-083273, filed May 11, 2020, and the entire disclosure of the Japanese Patent Applications are hereby incorporated by reference herein.

### [DESCRIPTION OF RELATED ART]

For example, in Patent Document 1, a toilet bowl in which a camera is arranged on the toilet bowl so as to capture a pooled water portion of the toilet bowl in which excrement is located is disclosed.

### [CITATION LIST]

### [Patent Document]

[Patent Document 1]
Japanese Unexamined Patent Application, First Publication No. 2007-252805

### [Summary of Invention]

### [Technical Problem]

In a conventional toilet seat device, measures for preventing a human body from being captured are insufficient. That is, there is a demand for a toilet seat device that physically realizes that the human body is not photobombed in the captured image such that the user can use the toilet seat device at ease, and there is room for improvement in that respect.

The present disclosure provides a toilet seat device configured to capture a target image in consideration of the privacy of the user such that the user can use at ease.

### [Solution to Solve Problem]

A toilet device according to an aspect of the present disclosure includes a toilet seat disposed on an upper side of a toilet; an image sensor provided on a back surface of the toilet seat and configured to image an inner space of a toilet bowl at time of excretion; an open-close acquiring unit configured to acquire open-close information of the toilet seat; and an imaging control unit configured to determine whether or not to image by the image sensor according to the open-close information of the toilet seat acquired by the open-close acquiring unit.

A toilet device according to another aspect of the present disclosure includes a toilet seat disposed on an upper side of a toilet; an image sensor provided individually from the toilet seat and on a lower side of the toilet seat, the image sensor being configured to image an inner space of a toilet bowl at the time of excretion; an open-close acquiring unit configured to acquire open-close information of the toilet seat; and an imaging control unit configured to determine whether or not to image by the image sensor according to the open-close information of the toilet seat acquired by the open-close acquiring unit.

### [BRIEF DESCRIPTION OF DRAWINGS]

Fig. 1 is a perspective view showing a toilet seat device according to a first embodiment of the present disclosure.
Fig. 2 is a plan view showing the toilet seat device shown in Fig. 1 viewed from an upper side.
Fig. 3 is a cross-sectional view taken along line A-A in Fig. 2.
Fig. 4 is a view showing a back surface of the toilet seat shown in Fig. 1.
Fig. 5 is a cross-sectional view taken along line B-B shown in Fig. 3.
Fig. 6 is a plan view showing a toilet of the toilet seat device viewed from the upper side.
Fig. 7 is a cross-sectional view taken along line C-C shown in Fig. 6.
Fig. 8 is a plan view of a camera cover.
Fig. 9 is a cross-sectional view taken along line D-D shown in Fig. 8.
Fig. 10 is a view showing an example of an image captured by an image sensor.
Fig. 11 is a vertical cross-sectional view of a toilet seat device according to a second embodiment.
Fig. 12 is a vertical cross-sectional view of a toilet seat device according to a third embodiment.
Fig. 13 is a vertical cross-sectional view of a toilet seat device according to a fourth embodiment.
Fig. 14 is a vertical cross-sectional view of a toilet seat device according to a fifth embodiment.
Fig. 15 is a vertical cross-sectional view of a toilet seat device according to a sixth embodiment.
Fig. 16 is a vertical cross-sectional view of a toilet seat device according to a first modification example.
Fig. 17 is a vertical cross-sectional view of a toilet seat device according to a second modification example.
Fig. 18 is a vertical cross-sectional view of a toilet seat device according to a seventh embodiment.
Fig. 19 is a vertical cross-sectional view showing a part of a toilet seat device according to an eighth embodiment.
Fig. 20 is a vertical cross-sectional view showing a part of a toilet seat device according to a ninth embodiment.
Fig. 21 is a vertical cross-sectional view showing a part of a toilet seat device according to a tenth embodiment.
Fig. 22 is a vertical cross-sectional view showing a part of a toilet seat device according to the tenth embodiment.

### [DETAILED DESCRIPTION OF EMBODIMENTS]

Hereinafter, various embodiments of a toilet seat device according to the present disclosure will be described referring to figures.

### (First Embodiment)

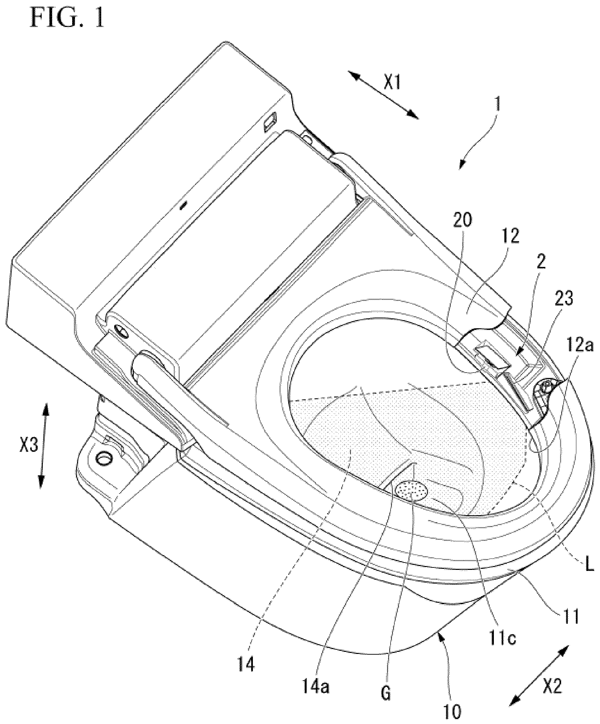

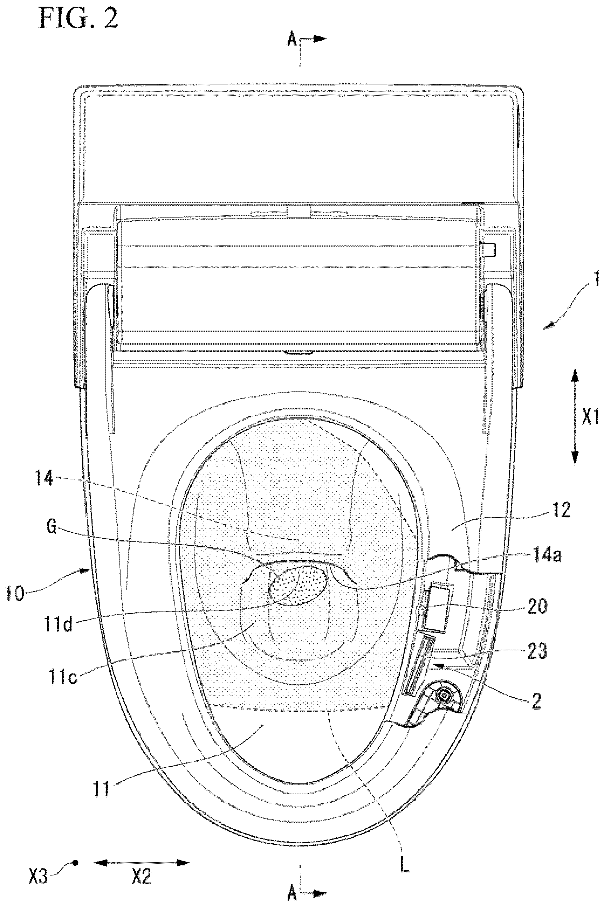

As shown in Fig. 1 and Fig. 2, a toilet seat device 1 according to the first embodiment includes a toilet 10, a toilet seat 12, a toilet lid, and an image determination device 2. The toilet 10 has a toilet bowl 11. The toilet seat 12 is provided to be openable and closable with respect to the toilet 10. The toilet lid is provided to be openable and closable with respect to the toilet seat 12. The toilet lid is not shown in figures. The image determination device 2 is configured to acquire an image of a stool G excreted by a user, and determines a state of the stool G or the like based on the acquired image.

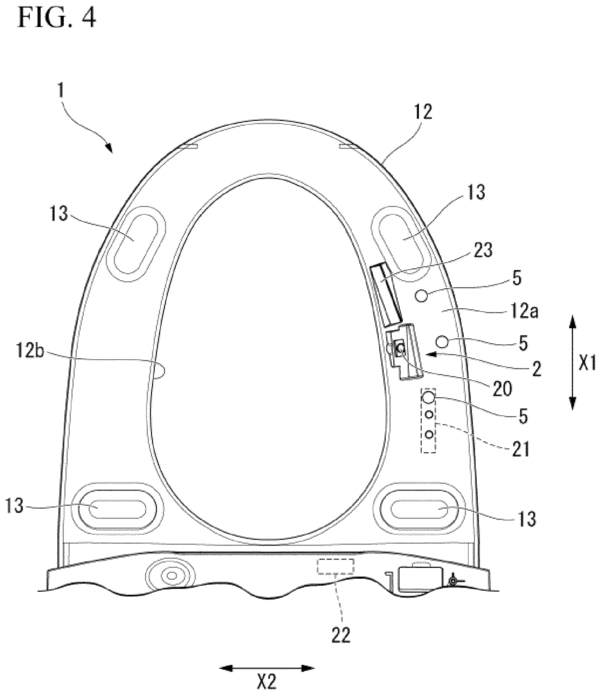

Fig. 1 is a perspective view of the toilet seat device 1 viewed from diagonally upper side thereof, and is a view in which a part of the toilet seat is broken. In Fig. 1, the toilet seat 12 in a closed state is shown. The toilet lid in the closed state covers an opening 11a of the toilet bowl 11 shown in Fig. 4 from the upper side with the toilet seat 12 sandwiched therebetween.

In the following description, a side in front of the user of the toilet seat device 1 is referred to as a front side in a state in which the user sits on the toilet seat 12. In the same state, a side behind the user is referred to as called the rear side. A direction connecting the front side and the rear side is referred to as a front-rear direction X1. A left side viewed from the user sitting on the toilet seat 12 is referred to as a "left side", and a right side is referred to as a "right side". A direction connecting the left side and the right side is referred to as a left-right direction X2. A side away from a floor surface on which the toilet seat device 1 is disposed is referred to as an "upper side", and a side closer to the floor surface is referred to as a "lower side". A direction connecting the upper side and the lower side is referred to as a vertical direction X3.

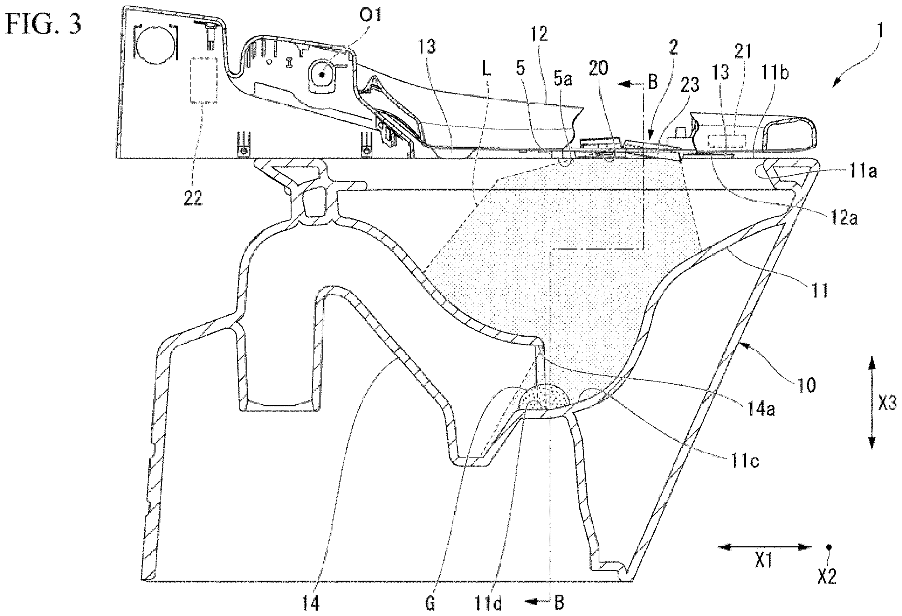

As shown in Fig. 3, a rim is provided on an upper surface 11b of the opening 11a of the toilet bowl 11. The rim projects toward an inner space of the toilet bowl 11. A pooled water portion 11c is arranged at a bottom portion of the toilet bowl 11. The pooled water portion 11c is connected to a toilet drainage channel 14 for discharging the stool G from the toilet bowl 11.

A rotation shaft O1 extending in the left-right direction X2 is provided at the rear portion of the toilet 10. The toilet seat 12 is configured to be freely rotatable around the rotation shaft 01. The toilet seat 12 is possible to enter either of a closed state or an open state by rotating as described above. In the closed state, the toilet seat 12 is in contact with an upper end portion of the toilet bowl 11 and extends in both the left-right direction X2 and the front-back direction X1. In the open state, the toilet seat 12 is away from the upper surface 11b of the toilet bowl 11 and extends at least in the vertical direction X3. The toilet seat 12 has an annular shape that extends continuously in a circumferential direction. The toilet seat 12 is in contact with the upper surface 11b of the toilet bowl 11 in the closed state. An opening size of the opening 12b of the toilet seat 12 is set to be slightly smaller than am opening size of the opening 11a of the toilet bowl 11 when viewed from the upper side. When the toilet seat 12 is in the closed state, the toilet seat 12 projects laterally from the upper surface 11b of the toilet bowl 11 toward an inner space.

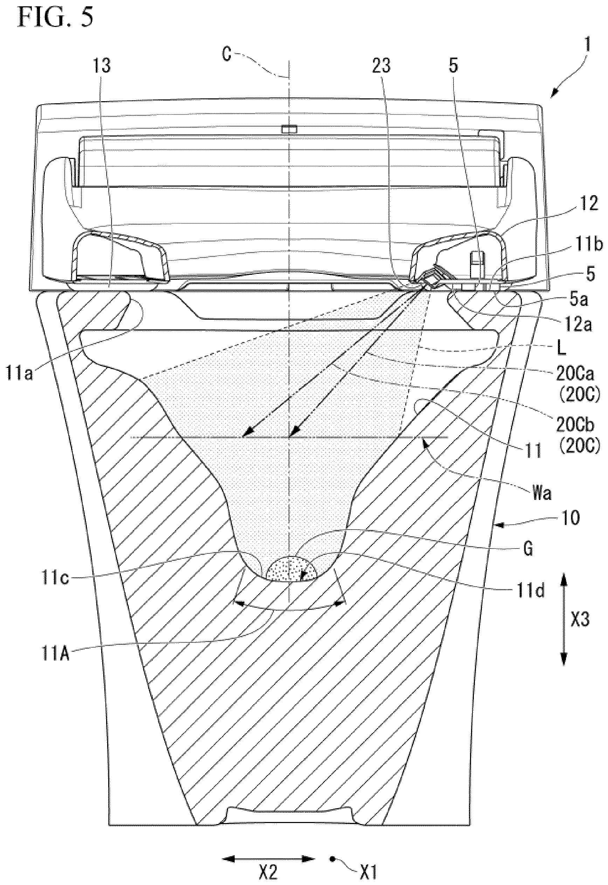

Fig. 4 is a view showing a back surface of the toilet seat 12 when the toilet seat 12 is in the open state. As shown in Fig. 4 and Fig. 5, the back surface 12a of the toilet seat 12 is provided with support members 13 projecting downward at four locations in the circumferential direction. The four support members 13 are configured to support the toilet seat 12 at four points with respect to the upper surface 11b of the toilet bowl 11 when the toilet seat 12 is in the closed state.

The toilet lid that is not shown in figures is configured to be freely rotatable around a rotation axis extending in the left-right direction X2. The toilet lid can enter either the closed state or the open state. In the closed state, the toilet lid covers the toilet seat 12 in the closed state and extends in the left-right direction X2 and the front-back direction X1 while closing the opening of each of the toilet bowl 11 and the toilet seat 12. In the open state, the toilet lid extends at least in the vertical direction X3. The toilet lid is provided so as to open and close the opening of the toilet bowl 11 and the opening of the toilet seat 12.

An image determination device 2 is provided on the back surface 12a of the toilet seat 12. The image determination device 2 includes an image sensor 20, an inclination sensor 21, an imaging control unit 22, and an illuminator 23. The image sensor 20 is provided on the back surface 12a of the toilet seat 12. The inclination sensor 21 acquires open-close information of the toilet seat 12. The inclination sensor 21 is an example of an open-close acquiring unit. The imaging control unit 22 controls the propriety of imaging by the image sensor 20 according to the open-close information of the toilet seat 12 acquired by the inclination sensor 21. The illuminator 23 emits light toward a deep side of the toilet drainage channel 14 such that the stool G that has moved from the bottom surface of the pooled water portion 11c of the toilet 10 toward the deep side, which is the downstream side of the toilet drainage channel 14, can be imaged.

The inclination sensor 21 is accommodated in the toilet seat 12. The inclination sensor 21 is set to detect that the toilet seat 12 has begun to open when the toilet seat 12 is opened by a certain angle or more. As an example, the inclination sensor 21 can employ a sensor in which a ball is accommodated inside such that the inclination of the toilet seat 12 is detected by the movement of the ball. The inclination sensor 21 is set to not to detect the inclination of the toilet seat 12 when the toilet seat 12 slightly rattles and lifts. The inclination sensor 21 is set to react when the toilet seat 12 is opened equal to or more than a predetermined angle. The open-close acquiring unit may use an automatic open-close device of the toilet seat 12 as the open-close sensor besides the inclination sensor 21.

In the present embodiment, the imaging control unit 22 is arranged on the toilet 10. The imaging control unit 22 is provided to be able to communicate wirelessly or by wire with the image sensor 20 and the inclination sensor 21. When the image sensor 22 determines that the toilet seat 12 is in the open state based on the open-close information acquired by the inclination sensor 21, the image sensor 20 includes the control of stopping the image sensor 20 from capturing the image of the internal space (pooled water portion 11c) of the toilet bowl 11 and the control capable of deleting the image captured when it is determined that the toilet seat 12 is in the open state. The imaging control unit 22 may be configured to interlock with a sitting sensor such as a load sensor or the like for detecting that the user is sitting on the toilet seat 12 and control the imaging of the internal space of the toilet bowl 11 by the image sensor 20 when the sitting is detected. The sitting sensor is not shown in the figures.

The image sensor 20 has a camera lens and an imaging device. The image sensor 20 is disposed on the back surface 12a of the toilet seat 12 and is arranged to face the toilet drainage channel opening 14a of the toilet drainage channel 14. The image sensor 20 is arranged such that an internal direction of the toilet drainage channel 14 to which the stool G is discharged from the pooled water portion 11c of the toilet bowl 11 can be imaged by the imaging device.

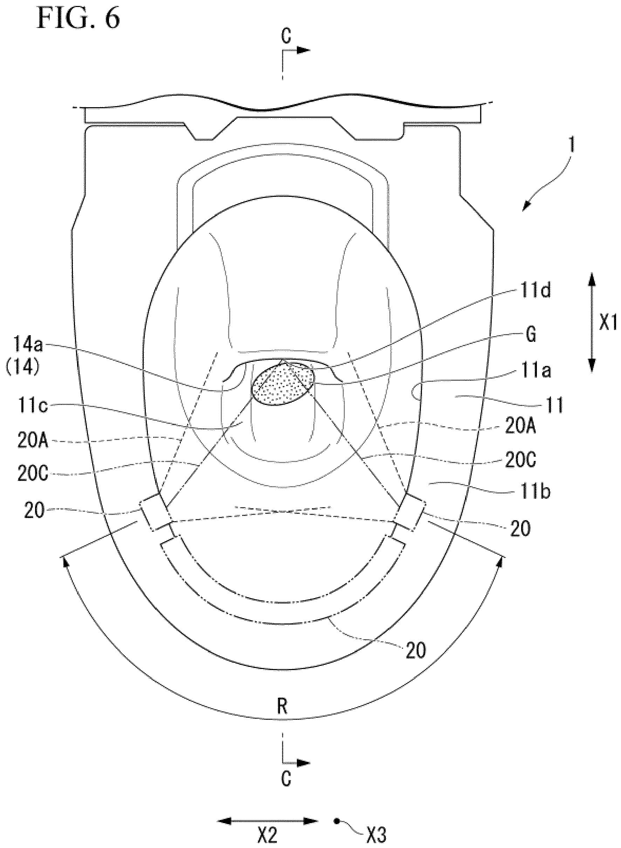

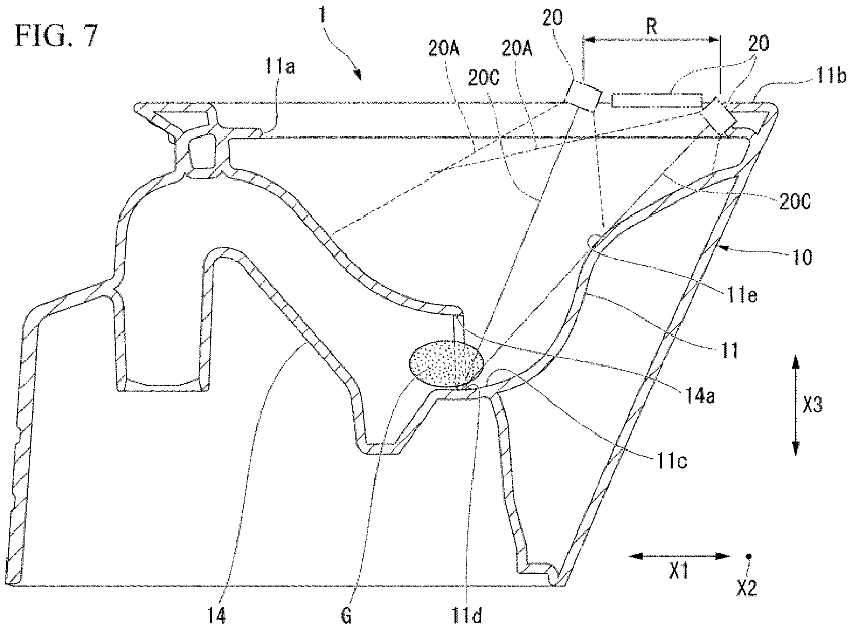

Fig. 6 is a plan view of the toilet 10 of the toilet seat device 1 as viewed from above. In Fig. 6, the toilet seat is not shown. As shown in Fig. 6 and Fig. 7, the image sensor 20 is arranged in front of the pooled water portion 11c. The image sensor 20 is arranged at a position where the deep side (rear side) of the toilet drainage channel opening 14a as an entrance of the toilet drainage channel 14 can be captured. A sensor arrangement region R of the image sensor 20 of the toilet seat device 1 is a range from the front region of the toilet seat to the lateral region of the toilet seat between the left side and the right side thereof, and is a left-right symmetrical region sandwiching the center of the left-right direction X2. The front region of the toilet seat is a region in which the stool G that has entered the toilet drainage channel opening 14a of the toilet bowl 11 can be easily seen. The lateral region of the toilet seat is a region where the opening 14a of the toilet drainage channel of the toilet bowl 11 can be peeped into, and it is possible to avoid the human body from being photobombed in a view angle (view field 20A) of the image sensor 20 in consideration of the psychological point of the user. The photobombing of the human body in the image means that in addition to the human body, an unintended object such as the buttocks, thighs, and male genitalia of the human body are captured in the image captured by the image sensor 20. In the photobombing of the human body in the image, in addition to a case in which the human body is directly imaged and captured, a case in which a state where the human body is reflected and imaged on the water surface Wa of the pooled water portion 11c is captured and the human body is imaged on the water surface Wa of the pooled water portion 11c in the captured image.

The image sensor 20 is arranged such that a line connecting the center of the camera lens and the lower end surface 11d of the opening of the toilet drainage channel opening 14a is a view field center 20C of the image sensor 20. At this time, it is preferable that the view field center 20C of the image sensor 20 is not shielded by the inner surface of the toilet bowl 11. For example, as shown in Fig. 7, the image sensor 20 located in front of the toilet seat 12 is arranged such that the view field center 20C does not intersect a convex curved surface 11e formed on the front inner surface of the toilet bowl 11. Here, the lower end surface 11d of the opening of the toilet drainage channel opening 14a is a predetermined region extending in the left-right direction X2 indicated by the reference sign 11A in the front view shown in Fig. 5.

As shown in Fig. 5, the image sensor 20 is preferably arranged such that the field view center 20C (20Ca, 20Cb) of the image sensor 20 faces toward the opposite position with respect of the image sensor by sandwiching the center line C of the pooled water portion 11c in the left-right direction X2 in the front view. Regarding the image sensor 20, the risk of the human body being reflected and imaged on the water surface Wa inside the toilet bowl 11 is reduced in a case of arranging the view field center along the arrow indicated by the reference sign 20Cb shown in Fig. 5 than a case of arranging the view field center along the arrow indicated by the reference sign 20Ca shown in Fig. 5. The image sensor 20 only has to be arranged in the direction of viewing the pooled water portion 11c from the position of the image sensor 20, and the view field center 20C of the image sensor 20 is directed to the distal side from the position of half of the water surface of the pooled water portion 11c in the toilet bowl 11.

The image sensor 20 has other functions besides the above-described functions. For example, it has a function of adjusting the brightness of the image according to the illuminance inside the toilet bowl 11.

The imaging control unit 22 may have a function of detecting dirt on the camera lens of the image sensor 20 from the image and prompting the user to clean the camera lens from the detected detection information.

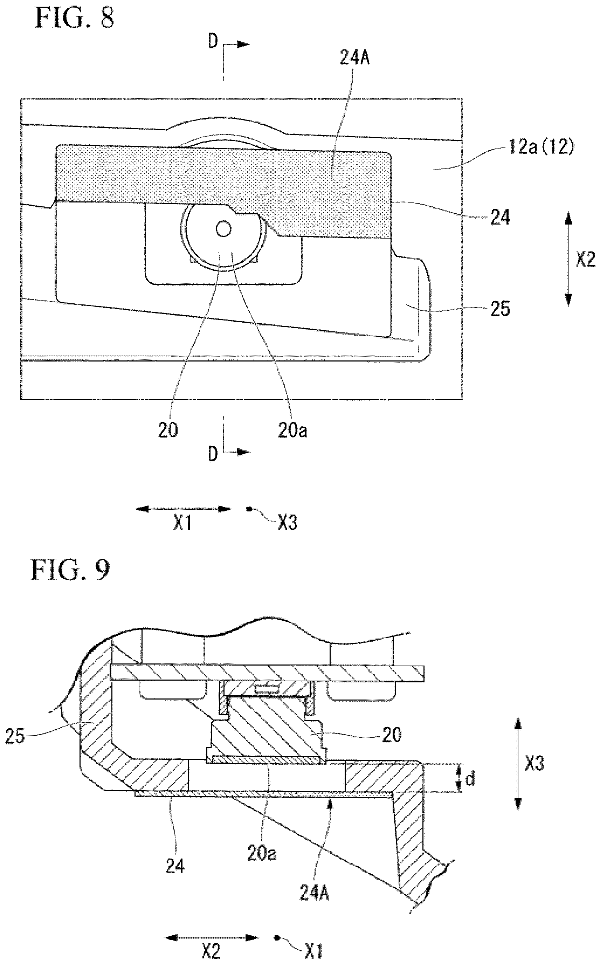

Fig. 8 is a plan view of a masking-printed camera cover 24 as viewed from the lower side. As shown in Fig. 8 and Fig. 9, a transparent camera cover 24 is provided in the image sensor 20. The camera cover 24 is arranged at a position at a predetermined distance d (see Fig. 9) from the camera lens in the imaging direction. The camera cover 24 physically masks a part of the human body, for example, a part in which the buttocks, thighs, male genitalia or the like are imaged, that is, a part of masking printing 24A described later. The camera cover 24 is arranged within a range of the view angle of the image sensor 20 between the image sensor 20 and the toilet bowl 11. The camera cover 24 is attached to the toilet seat 12a via a jig 25 fixed to the back surface 12a of the toilet seat 12. The camera cover 24 is printed or painted with an opaque masking print 24A in a predetermined region of the image in which a part of the human body is photobombed. The masking print 24A is an example of a shield.

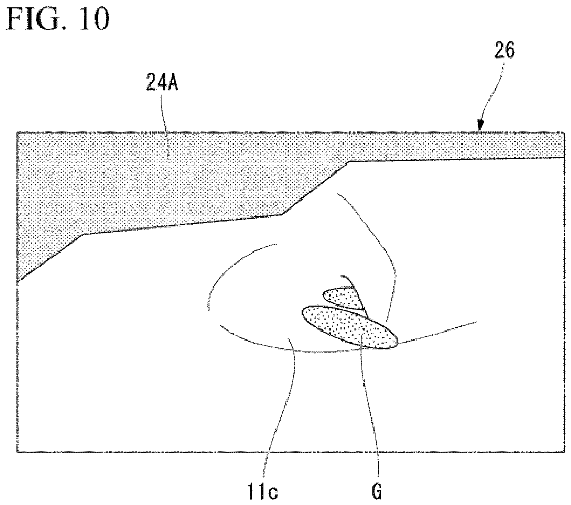

Fig. 10 is a view showing an example of an image captured by the image sensor 20 including a masking-printed camera cover 24. Fig. 10 is an image 26 captured by the image sensor 20, which is partially masked by the masking printing 24A. In this manner, it is possible to physically mask the region included in a part of the image and not intended to be imaged on the image.

As shown in Fig. 4, for example, an LED is used for the illuminator 23. The illuminator 23 is arranged on the back surface 12a of the toilet seat 12 and in front of the image sensor 20. As shown in Fig. 1, Fig. 2, Fig. 3, and Fig. 5, a range of the illumination L illuminated by the illuminator 23 is diffused from the light source such that the illumination center is located at the pooled water portion 11c of the toilet bowl 11. The illuminator 23 is arranged so as to substantially coincide with the position where the view field center 20C (see Fig. 5) of the image sensor 20 intersects the pooled water portion 11c as a target object. The positional relationship between the illuminator 23 and the image sensor 20 shown in the present embodiment is only an example. The illuminator 23 only has to be arranged near the image sensor 20 and at a position where the image sensor 20 can illuminate the deep side of the toilet drainage channel opening 14a.

As shown in Fig. 4, a plurality of protective legs 5 are provided on the back surface 12a of the toilet seat 12. The plurality of protective legs 5 are arranged in the vicinity of the image sensor 20. The plurality of protective legs 5 abut on the upper surface 11b of the toilet bowl 11 when the toilet seat 12 is in the closed state. The protective leg 5 is an example of a supporter. Fig. 4 shows an example having three protective legs 5. The protective leg 5 is made of, for example, a rubber member. As shown in Fig. 3 and Fig. 5, the protective leg 5 projects downward from the back surface 12a of the toilet seat 12 when the toilet seat 12 is closed, and a protruding end 5a of the protective leg 5 abuts on the upper surface 11b of the toilet bowl 11. When the toilet seat 12 is in the closed state, the toilet seat 12 is supported by the lower toilet bowl 11 by the four support members 13 and the three protective legs 5. The three protective legs 5 are arranged on the outer peripheral side with respect to the image sensor 20 located on the inner peripheral side of the toilet seat 12, and are arranged at intervals in the circumferential direction.

The protective leg 5 projects at a height such that the image sensor 20 arranged on the back surface 12a of the toilet seat 12 does not come into contact with the upper surface 11b of the toilet bowl 11 when the toilet seat 12 is in the closed state. For example, the protruding length of the protective leg 5 is longer than the length of the image sensor 20 protruding from the back surface 12a of the toilet seat 12. For example, in a case in which there is a portion in the image sensor storage portion protruding from the back surface 12a of the toilet seat 12, the protective leg 5 is provided to be projects such that the image sensor storage portion does not abut on the upper surface 11b of the toilet bowl 11. Alternatively, the protective leg 5 may not be provided and the supporter 13 may be provided such that a clearance with the upper surface 11b of the toilet bowl 11 formed by the supporter 13 of the toilet seat 12 is secured to be equal to or larger than the height at which the image sensor storage portion projects from the back surface 12a of the toilet seat 12.

Accordingly, it is possible to prevent any external contact from being applied to the image sensor 20 even in the closed state by the protective leg 5 or the supporter 13 of the toilet seat 12 disposed in the vicinity of the image sensor 20. For example, when the toilet seat 12 is in the closed state, there is a case in which the toilet seat 12 is deformed due to the load of the user sitting thereon. Even in such a situation, by the protective leg 5 or the supporter 13, it is possible to prevent the contact of the image sensor 20 with respect to the upper surface 11b of the toilet bowl 11 due to the deformation of the toilet seat 12.

Next, the effects of the toilet seat device 1 according to the present embodiment will be described in details based on the figures. As shown in Fig. 3 and Fig. 4, in the toilet seat device 1 according to the present embodiment, only when the imaging control unit determines that it is possible to image according to the open-close information of the toilet seat 12 acquired by the inclination sensor 21, it is possible for the image sensor 20 to image the internal space of the toilet bowl 11. That is, when the imaging control unit 22 determines that it is impossible to image, the imaging by the image sensor 20 is physically stopped. Accordingly, for example, in the state in which the image is impossible at the time of the opening and closing of the toilet seat 12, it is possible to definitely prevent the human body from being unintentionally captured by the image sensor 20 provided on the back surface 12a of the toilet seat 12. Accordingly, in the toilet seat device 1 according to the present embodiment, the user can use at ease by capturing the target image in consideration of the privacy of the user.

In the present embodiment, the imaging control unit 22 controls the image sensor 20 to capture the image of the internal space of the toilet bowl 11 when it is determined that the toilet seat 12 is in the closed state based on the open-close information acquired by the inclination sensor 21. In this case, for example, when the toilet seat 12 is opened at a predetermined angle or more, the imaging by the image sensor 20 is stopped such that the imaging is controlled to be performed only when the user sits on the toilet seat 12 and excretes. Therefore, the imaging is not performed before and after the user sitting on the toilet seat, and the user can use the toilet seat device 1 at ease. The imaging control unit 22 may set whether or not to perform the imaging by the image sensor 20 according to the open-close angle of the inclination sensor 21. Therefore, for example, the imaging control unit 22 may perform the control such that the toilet seat 12 is not determined to be in the open state even if the toilet seat 12 is slightly opened or displaced within a range in which the human body is not photobombed in the captured image.

In the present embodiment, the image sensor 20 is disposed on the back surface 12a of the toilet seat 12 and may image the internal direction of the toilet drainage channel 14 for discharging the stool G from the pooled water portion 11c of the toilet bowl 11, and the image sensor 20 is arranged to face the direction toward the toilet drainage channel opening 14a. In this case, the stool G that has rolled into the deep side of the toilet drainage channel opening 14a in the toilet bowl 11 does not become a blind spot for imaging, and the entire excreted stool G can be definitely imaged.

In the present embodiment, since the illuminator 23 can irradiate the deep side of the toilet bowl 11 from the bottom surface of the pooled water portion 11c of the toilet drainage channel 14, the image sensor 20 may image the stool G more clearly.

In the present embodiment, the image sensor 20 is arranged at a position where the human body is not photobombed in the view field 20A of the image sensor 20. In this case, as described above, it is possible to prevent the human body from being photobombed in the view field 20A imaged by the image sensor 20 when the imaging control unit 22 determines that the imaging may be performed.

In the present embodiment, since the image sensor 20 is configured such that the image can be adjusted to the brightness according to the illuminance inside the toilet bowl 11, it is possible to image the stool G more clearly by the image sensor 20.

In the present embodiment, the camera cover 24 is arranged between the image sensor 20 and the toilet bowl 11, and there is a configuration that the masking printing 24A is printed on the camera cover 24 in the region where the human body is photobombed in the range of the view angle (view field 20A) of the image sensor 20. In this case, it is possible to physically prevent the photobombing of the human body in the captured image without adjusting the view angle of the image sensor 20. According to such a configuration, there is an advantage that the region of the masking printing 24A can be easily changed according to the position of the image sensor 20 and the shape of the toilet 10.

The toilet seat device 1 according to the present embodiment has a function of detecting a dirt on the camera lens 20a of the image sensor 20 from the image captured by the image sensor 20 and outputting a cleaning-requirement signal. The cleaning-requirement signal is a signal meaning that the camera lens 20a needs to be cleaned. Based on the cleaning-requirement signal, a sign for requiring cleaning is displayed on a display that is not shown in the figures. In this case, the camera lens 20a may be cleaned when the user recognizes the sign that cleaning is required. Therefore, it is possible to always acquire the image easy to see, and it is possible to prevent a problem that it becomes difficult to determine the stool G being captured in image due to the dirt on the camera lens 20a of the image sensor 20.

In the present embodiment, the protective legs 5 are provided on the back surface 12a of the toilet seat 12 so as to come into contact with the upper surface 11b of the toilet bowl 11 when the toilet seat 12 is in the closed state. Even if the toilet seat 12 bends due to the weight of the user when the user sits on the toilet seat, the protective legs 5 support the toilet seat 12 so as to prevent the position of the toilet seat 12 from being significantly displaced. As a result, it is possible to prevent the imaging range of the image sensor 20 from being shifted and to prevent the image sensor 20 from being damaged due to stress from the toilet seat 12.

The toilet seat device 1 according to the present embodiment captures the target image in consideration of the privacy of the user, such that the user can use it at ease.

### (Second Embodiment)

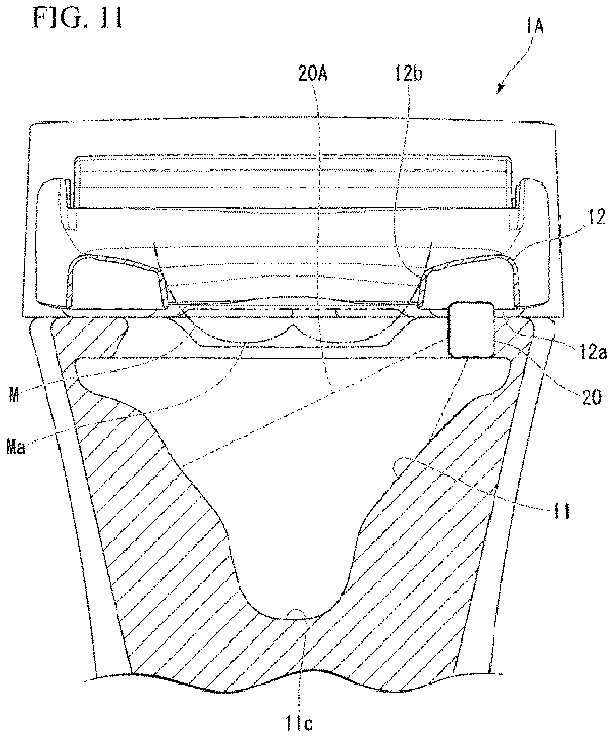

Fig. 11 shows a vertical cross section of the toilet seat device 1A according to the second embodiment as viewed from the front. As shown in Fig. 11, the toilet seat device 1A according to the second embodiment has a configuration in which the image sensor 20 is arranged at a position separated from the back surface 12a of the toilet seat 12 toward the toilet bowl 11 (downward). For example, the image sensor 20 may have a configuration in which the image sensor 20 is attached to a spacer (not shown) that functions as a sensor fixer having an arbitrary shape provided on the back surface 12a of the toilet seat 12. The sensor fixing portion is an example of a photobomb preventer. For example, the image sensor 20 may be provided integrally with the toilet seat 12 in a shape protruding downward from the lower surface of the toilet seat 12.

In the second embodiment, since the image sensor 20 is provided to project downward at the inside of the toilet bowl 11, even in a case in which the buttock M of the human body sitting on the toilet seat 12 is located below the back surface 12a of the toilet seat 12, the image sensor 20 may be positioned below the lower surface Ma of the buttock M. Therefore, it is possible to realize a configuration in which the human body is not physically in the view field of the image sensor 20. In the second embodiment, it is possible to prevent the human body from being photobombed in the image captured by the image sensor 20, and by capturing the target image in consideration of the privacy of the user, the user can use it at ease.

### (Third Embodiment)

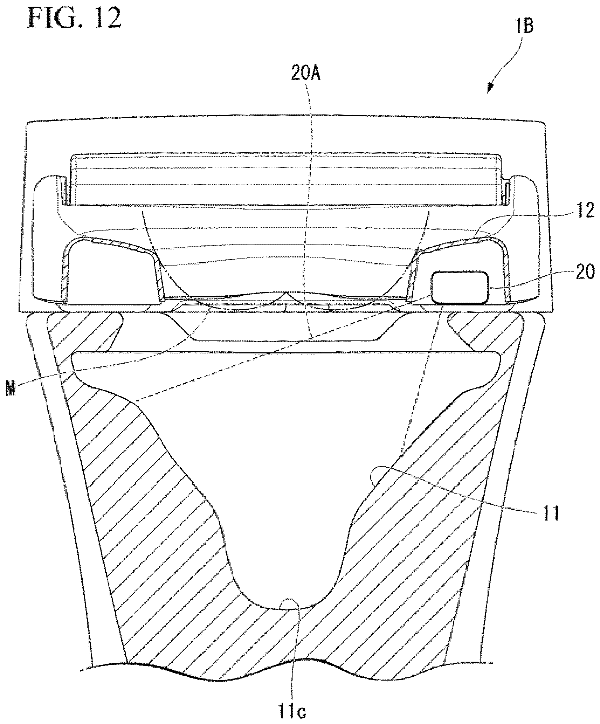

Fig. 12 is a view showing a vertical cross section of a toilet seat device 1B according to a third embodiment as viewed from the front. As shown in Fig. 12, the toilet seat device 1B according to the third embodiment has a portion for increasing the thickness of the toilet seat 12, and a size thereof is set such that the buttocks M of the person sitting on the seat does not fall below the back surface 12a of the toilet seat 12. The portion for increasing the thickness of the toilet seat 12 is an example of the photobomb preventer. The position of the image sensor 20 in this case is the same as that of the first embodiment described above, and the image sensor 20 is directly provided on the back surface 12a of the toilet seat 12.

In the third embodiment, since the thickness of the toilet seat 12 itself is increased, the toilet seat 12 has a structure that may physically prevent the buttocks M of the human body sitting on the toilet seat 12 from being located below the back surface 12a of the toilet seat 12. In the third embodiment, it is possible to prevent the human body from being photobombed in the image captured by the image sensor 20, and the user can use at ease since the target image is captured in consideration of the privacy of the user.

### (Fourth Embodiment)

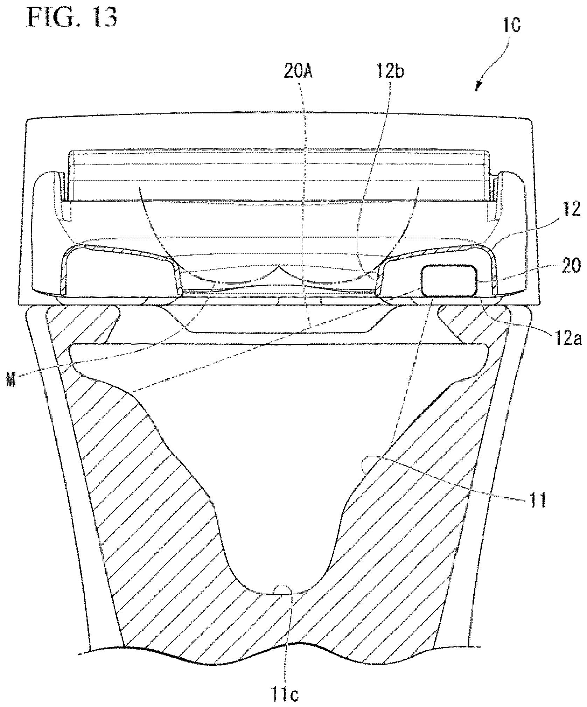

Fig. 13 shows a vertical cross section of a toilet seat device 1C according to a fourth embodiment as viewed from the front. As shown in Fig. 13, the toilet seat device 1C according to the fourth embodiment has an opening reduction portion in which a radial dimension of the opening 12b of the toilet seat 12, particularly the length in the left-right direction X2, is reduced, and has a configuration with a shape set such that the buttock portion M of the person sitting on the seat does not fall below the back surface 12a of the toilet seat 12. The opening reduction portion is an example of the photobomb preventer. The position of the image sensor 20 in this case is the same as that of the first embodiment described above, and is directly provided on the back surface 12a of the toilet seat 12.

In the fourth embodiment, since the opening 12b of the toilet seat 12 becomes smaller, the toilet seat 12 has a structure for physically preventing the buttocks M of the human body sitting on the toilet seat 12 from being located below the back surface 12a of the toilet seat 12. In the fourth embodiment, it is possible to prevent the human body from being photobombed in the image captured by the image sensor 20, and the user can use at ease since the target image is captured in consideration of the privacy of the user.

### (Fifth Embodiment)

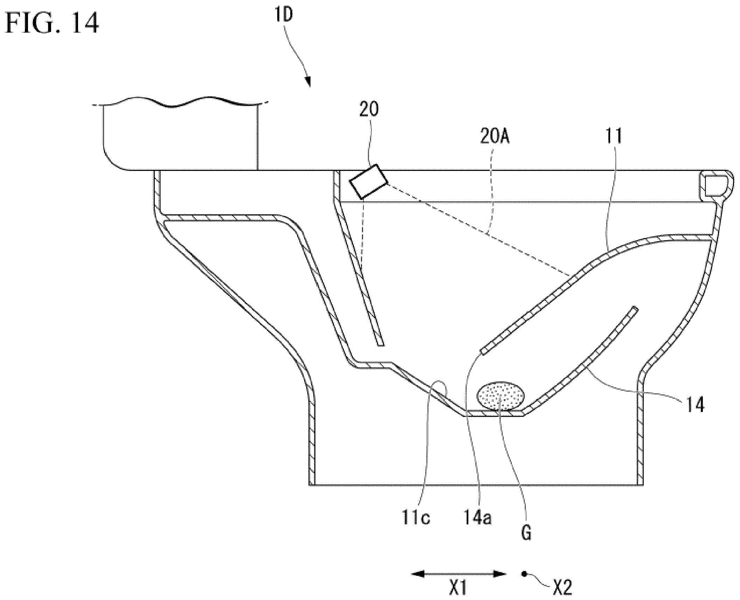

Fig. 14 shows a cross section of a toilet seat device 1D according to a fifth embodiment at the center in the left-right direction and along the front-rear direction. In the above-described embodiment, the inclination sensor 21 accommodated in the toilet seat 12 is adopted as the open-close acquiring unit for detecting the open-close of the toilet seat 12, and the open-close information is obtained by the inclination angle of the toilet seat 12. The configuration of the open-close acquiring unit is not limited to this example. For example, the fifth embodiment is an example in which the open-close acquiring unit is adopted as shown in Fig. 14. In the fifth embodiment, as another open-close acquiring unit of the toilet seat 12, a sitting sensor (not shown) for detecting when the user is sitting on the toilet seat 12 is provided, and the sitting sensor may be configured to control the image sensor 20 to image the internal space of the toilet bowl 11 only when it is determined that the user is sitting on the seat. For example, the sitting sensor may adopt a load sensor or the like.

### (Sixth Embodiment)

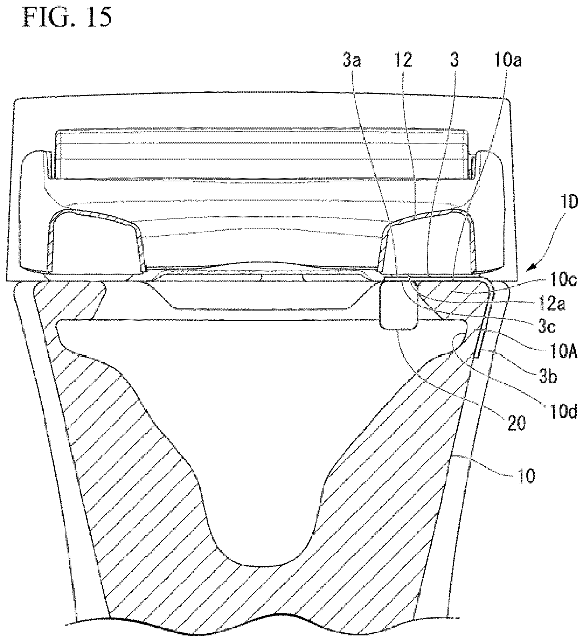

Fig. 15 shows a vertical cross section of a toilet seat device 1D according to a sixth embodiment as viewed from the front. As shown in Fig. 15, the toilet seat device 1D according to the sixth embodiment has a configuration in which the image sensor 20 is separately provided below the toilet seat 12. In the sixth embodiment, a region where the image sensor 20 is provided, that is, the region separated from the toilet seat 12 and below the toilet seat 12 is included in the back surface of the toilet seat 12. That is, the back surface of the toilet seat 12 also includes the lower region of the toilet seat 12. The toilet seat device 1D is provided with a connecting bar 3 formed of, for example, a metal member. The connecting bar 3 is provided on the upper surface 10a of the toilet 10 to bridge between the inside of the toilet and the outside of the toilet. The image sensor 20 is attached to a lower surface 3c of an inner-toilet end portion 3a of the connecting bar 3 so as to image the internal space of the toilet 10. An outer-toilet end portion 3b of the connecting bar 3 is attached to an outer peripheral surface of the toilet 10 by sticking or suctioning. The image sensor 20 is located below the toilet seat 12 and on the lateral side of the rim 10A of the toilet 10. Specifically, the image sensor 20 is arranged on the lateral side of the rim 10A from a position at the same height as the upper surface 10a of the toilet bowl to the lateral side of an upper rim 10c and a standing surface 10d of the rim 10A. The image sensor 20 may be a sensor including the image sensor 20.

In the connecting bar 3, a band-shaped member extending in one direction is bent in an L shape from a toilet upper surface 10a along the outer shape of the outer peripheral surface. The inner-toilet end portion 3a of the connecting bar 3 projects from the toilet upper surface 10a toward the inside of the toilet 10. The outer-toilet end portion 3b is fixed to the toilet 10 with an adhesive. The means for fixing the connecting bar 3 to the toilet 10 may be a suction cup instead of an adhesive. In the case of using the suction cup, it is easy to attach to and detach from the toilet 10 such that an effect of making cleaning and maintenance to be easy may be realized, and further, the position of the image sensor 20 may be easily adjusted.

### (First Modification Example)

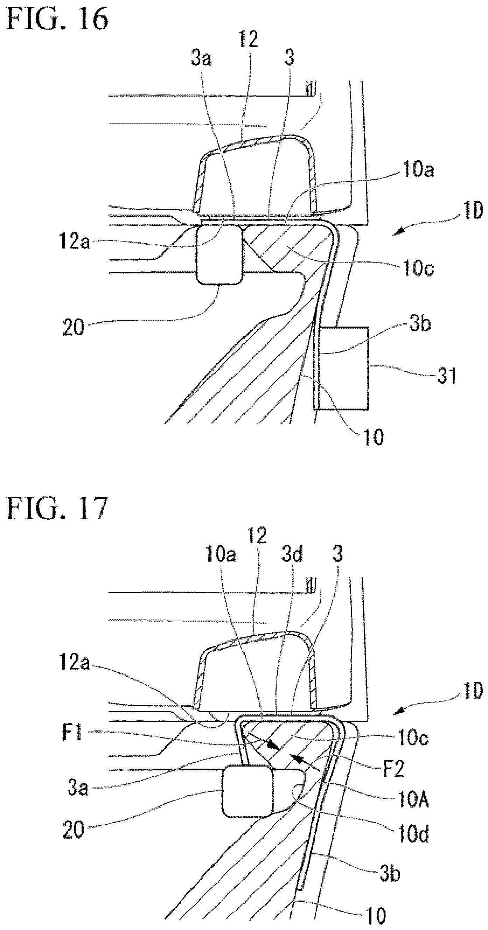

Fig. 16 shows a vertical cross section of a part of a toilet seat device of the first modification example of the sixth embodiment as viewed from the front. In the first modification example of the sixth embodiment, as shown in Fig. 16, a weight 31 for maintaining a load balance with the image sensor 20 provided at the inner-toilet end portion 3a is attached to the outer-toilet end portion 3b of the connecting bar 3. The weight 31 is fixed on a surface facing the outside of the outer-toilet end portion 3b opposite to the toilet 10 side, for example, with an adhesive or the like. The weight 31 may be, for example, a metal member. The weight 31 may be, for example, a camera functional part that functions as an image processor or the like that processes data of the image sensor 20. In the first modification example, the image sensor 20 is attached to the toilet 10 in a state in which the image sensor 20 is positioned in a balanced manner by the weight of the weight 31 of the connecting bar 3, and it is possible to prevent the image sensor 20 from inclining or shifting.

### (Second Modification Example)

Fig. 17 shows a vertical cross section of a part of a toilet seat device of a second modification example of the sixth embodiment as viewed from the front. As shown in Fig. 17, the connecting bar 3 of the second modification example is formed of a metal wire, a metal plate or the like. The connecting bar 3 is bent into a substantially U shape. The connecting bar 3 has a central portion 3d in the length direction that is disposed on the toilet upper surface 10a and has a configuration in which an elastic force is generated in a direction (the arrow F1 and arrow F2 shown in Fig. 17) in which the inner-toilet end portion 3a and the outer-toilet end portion 3b being opposite to each other approach each other. The image sensor 20 is attached to the inner-toilet end portion 3a. The connecting bar 3 is attached thereto to sandwich the upper portion of the toilet 10 such that the inner-toilet end portion 3a and the outer-toilet end portion 3b are fitted onto the upper portion of the toilet 10 from the outside in a state in which the inner-toilet end portion 3a and the outer-toilet end portion 3b are in an open state against the force along the direction (the arrow F1 and arrow F2 shown in Fig. 17) in which the inner-toilet end portion 3a and the outer-toilet end portion 3b being opposite to each other approach each other. In the first modification example, the image sensor 20 is attached to the toilet 10 in the state in which the image sensor 20 is positioned in the balanced manner due to the weight of the weight 31 of the connecting bar 3, and it is possible to prevent the image sensor 20 from inclining and shifting. The image sensor 20 is arranged on the lateral side of the rim 10A below the back surface 12a of the toilet seat 12. Specifically, the image sensor 20 is arranged on the lateral side of the rim 10A at a position at a height below the upper rim 10c and on the lateral side of the standing surface 10d of the rim 10A. Here, as the definition of the back surface 12a of the toilet seat 12, the region may include the region below the toilet seat 12.

### (Seventh Embodiment)

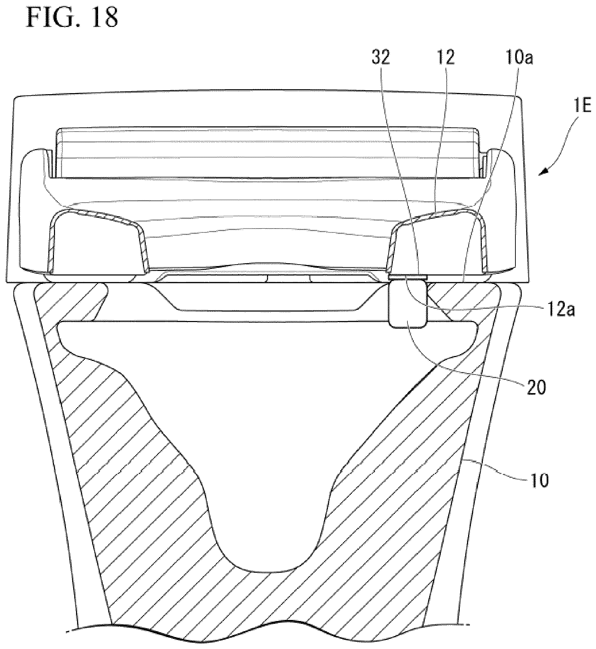

Fig. 18 shows a vertical cross section of a toilet seat device 1E according to a seventh embodiment as viewed from the front. As shown in Fig. 18, the toilet seat device 1E according to the seventh embodiment has a configuration in which the image sensor 20 is directly fixed to the back surface 12a of the toilet seat 12 via a fixing portion 32 such as an adhesive or the like. An attachment position of the image sensor 20 with respect to the toilet seat 12 is a position closer to an inner circumference of the toilet seat 12, and the image sensor 20 attached when the toilet seat 12 is in the closed state (the state shown in Fig. 18) is disposed at a position not to interfere with the upper portion of the toilet 10. According to the seventh embodiment, the structure is only configured to fix the image sensor 20 to the toilet seat 12. Accordingly, the toilet seat device 1E does not require a separate member such as the connecting bar 3 (see Fig. 15) of the sixth embodiment described above, and there is an advantage that the image sensor 20 may be easily attached to reduce the member cost.

### (Eighth Embodiment)

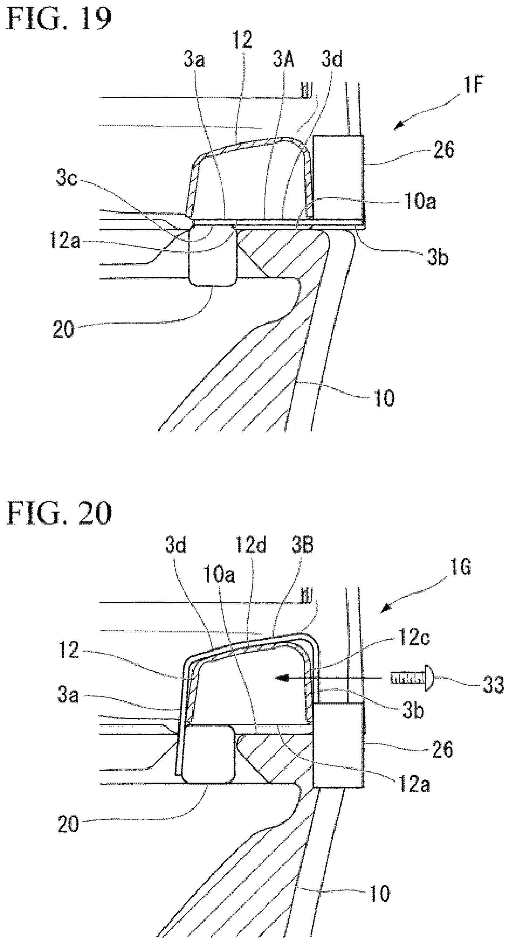

Fig. 19 shows a vertical cross section of a part of a toilet seat device 1F according to an eighth embodiment as viewed from the front. As shown in Fig. 19, the toilet seat device 1F according to the eighth embodiment is an example in which the image sensor 20 is attached to the toilet seat 12 via a second connecting bar 3A. The second connecting bar 3A is formed of, for example, a metal wire or a metal plate. The second connecting bar 3A extends between the back surface 12a of the toilet seat 12 and the outside of the toilet seat 12. The image sensor 20 is attached to the lower surface 3c of the inner-toilet end portion 3a of the second connecting bar 3A so as to image the internal space of the toilet 10. The outer-toilet end portion 3b of the second connecting bar 3A projects outward of the toilet seat 12. A camera functional part 26 that is electrically connected to the image sensor 20 is provided in the outer-toilet end portion 30b. The camera functional part 26 is arranged along the outer side surface 12c of the toilet seat 12. The central portion 3d of the second connecting bar 3A is fixed to the back surface 12a of the toilet seat 12 by a fixing means such as an adhesive, a suction cup or the like. The camera functional part 26 has a function such as an image processer configured to process the data of the image sensor 20.

In the eighth embodiment, the image sensor 20 may be retrofitted to the toilet seat 12 side via the second connecting bar 3A. Therefore, a simpler structure than the case of attaching the image sensor 20 to the toilet 10 may be adopted.

### (Ninth Embodiment)

Fig. 20 shows a vertical cross section of a part of a toilet seat device 1G according to a ninth embodiment as viewed from the front. As shown in Fig. 20, the toilet seat device 1G according to the ninth embodiment is an example in which the image sensor 20 is attached to the toilet seat 12 via a third connecting bar 3B. The third connecting bar 3B is formed of, for example, a metal wire or a metal plate. The third connecting bar 3B is bent into a substantially U shape to bridge the upper side of the toilet seat 12. The connecting bar 3 has a central portion 3d in the length direction disposed on the toilet seat 12. The image sensor 20 is attached to the inner-toilet end portion 3a of the third connecting bar 3A so as to image the internal space of the toilet 10. The image sensor 20 attached to the third connecting bar 3A is provided to be located on the back surface 12a of the toilet seat 12.

The camera functional part 26 is provided on the outer-toilet end portion 3b of the third connecting bar 3A along the outer surface 12c of the toilet seat 12. The camera functional part 26 is electrically connected to the image sensor 20. The third connecting bar 3B is fixed by pressing the outer-toilet end portion 3b against the outer side surface 12c of the toilet seat 12 by a pressing member 33 such as a screw or the like pressing from the outside of the outer-toilet end portion 3b. The method of fixing the third connecting bar 3 to the toilet seat 12 is not limited to the pressing member 33 described above, and other fixing method may be adopted. For example, the central portion 3d of the third connecting bar 3B may be fixed to the upper surface 12d of the toilet seat 12 by a fixing means such as an adhesive, a suction cup or the like. Alternatively, the third connecting bar 3B may be attached to the toilet seat 12 by sandwiching the toilet seat 12 from two sides such that an elastic force is generated in the direction in which the inner-toilet end portion 3a and the outer-toilet end portion 3b facing each other approach each other.

### (Tenth Embodiment)

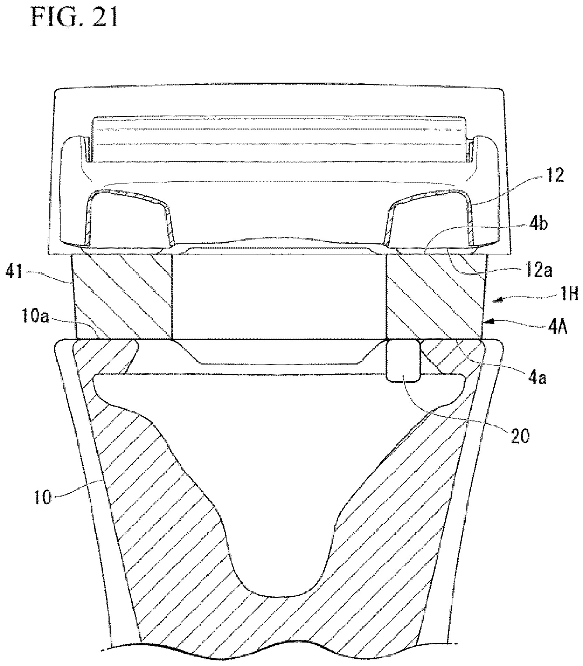

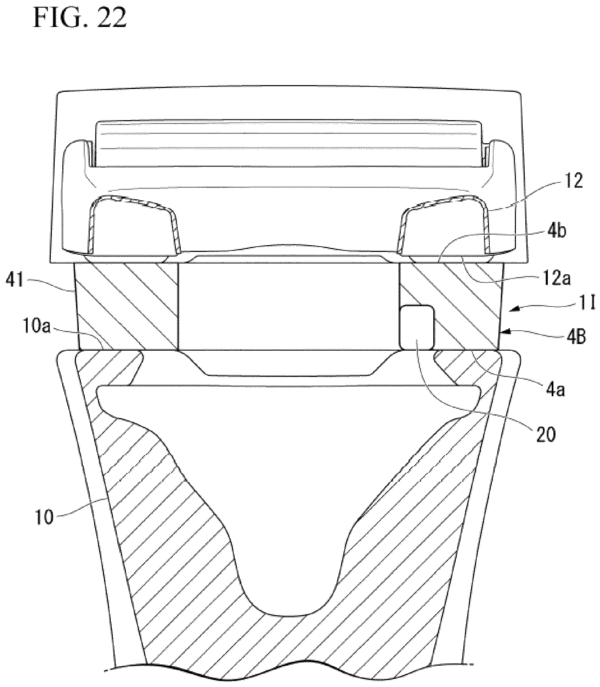

Fig. 21 and Fig. 22 show a vertical cross section of a part of a toilet seat device 1H according to a tenth embodiment as viewed from the front. As shown in Fig. 21 and Fig. 22, the toilet seat device 1H and a toilet seat device 1I according to the tenth embodiment have a structure in which separate toilet seat 4A and toilet seat 4B are sandwiched between the toilet seat 12 and the toilet upper surface 10a. In the first toilet seat 4A shown in Fig. 21, the image sensor 20 is fixed to a lower surface 41a of a toilet-seat-type insertion member 41. As shown in Fig. 22, in the second toilet seat 4B, the image sensor 20 is built in the toilet-seat-type insertion member 41. The toilet-seat-type insertion member 41 is formed to have substantially the same shape as the toilet seat 12 in a plan view, and is provided to open and close with respect to the upper surface 10a together with the toilet seat 12 or individually from the toilet seat 12. In a state in which the toilet-seat-type insertion member 41 is in the closed state, the lower surface 41a of the toilet-seat-type insertion member 41 is disposed on the toilet upper surface 10a, and the upper surface 41b of the toilet-seat-type insertion member 41 is in contact with the back surface 12a of the toilet seat 12. The image sensor 20 is attached so as to image the internal space of the toilet 10.

Although the embodiments of the toilet seat device according to the present disclosure have been described above, the present disclosure is not limited to the above-described embodiments.

For example, in the above-described embodiments, an image processor configured to perform image processing on the target image acquired by the image sensor 20 may be provided in the image determination device 2. In this case, in the image processor, the process of masking, hiding, or erasing the region in which the human body is photobombed in the target image may be performed.

In the above-described embodiments, an example of the toilet seat device 1 in which the toilet drainage channel 14 for draining water from the pooled water portion 11c of the toilet bowl 11 toward the rear side of the toilet 10 and the image sensor 20 is arranged in front of the toilet seat 12 is shown. The toilet seat device is not limited to such a toilet structure, and may be a toilet seat device having another structure. For example, as the toilet seat device 1D according to the fifth embodiment shown in Fig. 14, the configuration in which the toilet drainage channel 14 for draining water from the pooled water portion 11c toward the front side of the toilet 10 is provided, and the image sensor 20 is arranged at the rear side of the toilet may be realized.

In the present embodiment, in order to protect the image sensor 20, the configuration in which the protective legs 5 are provided on the back surface 12a of the toilet seat 12 as supporters to be in contact with the upper surface 11b of the toilet bowl 11 when the toilet seat 12 is in the closed state, is disclosed as example. However, the toilet seat is not limited to the configuration in which the protective legs 5 are provided, and the protective legs 5 may be omitted. The supporter is not limited to the configuration such as the shape and amount of the protective legs 5 according to the above-described embodiment, and can be appropriately set.

In addition, it is possible to suitably replace the components in the above-described embodiment with well-known components without departing from the spirit of the scope of the present disclosure.

### [INDUSTRIAL APPLICABILITY]

According to the toilet seat device of the present disclosure, the user can use the toilet seat device at ease by capturing the target image in consideration of the privacy of the user.

### [REFERENCE SIGNS LIST]

1, 1A, 1B, 1C, 1D, 1E, 1F, 1G, 1H, 1I toilet seat device
2 image determination device
5 protective leg (supporter)
10 toilet
10A rim
11 toilet bowl
11d lower end surface of opening
12 toilet seat
12a back surface
13 supporter
14 toilet drainage channel
14a toilet drainage channel opening
20 image sensor
20A view field
20C view field center
21 inclination sensor (open-close acquiring unit)
22 imaging control unit
23 illuminator
24 camera cover
24A masking printing (shield)
G stool
X1 front-back direction
X2 left-right direction
X3 up-down direction

## Claims

1. A toilet device, comprising:
a toilet seat disposed on an upper side of a toilet;
an image sensor provided on a back surface of the toilet seat and configured to image an inner space of a toilet bowl at time of excretion;
an open-close acquiring unit configured to acquire open-close information of the toilet seat; and
an imaging control unit configured to determine whether or not to image by the image sensor according to the open-close information of the toilet seat acquired by the open-close acquiring unit.

2. A toilet device, comprising:
a toilet seat disposed on an upper side of a toilet;
an image sensor provided individually from the toilet seat and on a lower side of the toilet seat, the image sensor being configured to image an inner space of a toilet bowl at the time of excretion;
an open-close acquiring unit configured to acquire open-close information of the toilet seat; and
an imaging control unit configured to determine whether or not to image by the image sensor according to the open-close information of the toilet seat acquired by the open-close acquiring unit.

3. The toilet device according to claims 1 or 2, wherein the imaging control unit is configured to:
control to stop imaging of the internal space of the toilet bowl by the image sensor when the toilet seat is determined to be in an open state according to the open-close information acquired by the open-close acquiring unit, and
control to delete a captured image when the toilet seat is determined to be in the open state.

4. The toilet device according to claims 1 or 2,
wherein the open-close acquiring unit is a sitting sensor configured to detect that a user has sat on the toilet seat, and
the imaging control unit is configured to control to image the internal space of the toilet bowl by the image sensor when sitting information is detected by the sitting sensor.

5. The toilet device according to any one of claims 1 to 4,
wherein the image sensor is disposed to direct to a direction facing an opening of a drainage channel so as to be able to image an internal direction of the drainage channel to which stool is discharged from a pooled water portion of the toilet.

6. The toilet device according to claim 5, further comprising an illuminator configured to illuminate a deep side of a bottom surface of the pooled water portion of the toilet.

7. The toilet device according to any one of claims 1 to 6,
wherein the image sensor is disposed at a position where a human body is not photobombed in a view field of the image sensor.

8. The toilet device according to any one of claims 1 to 7,
wherein the image sensor is disposed at a position separated from a back surface of the toilet seat in a direction toward the toilet bowl.

9. The toilet device according to any one of claims 1 to 8,
wherein the image sensor is adjustable to a brightness corresponding to an illuminance at an inner side of the toilet bowl.

10. The toilet device according to any one of claims 1 to 9,
wherein a shield is disposed in a range of a view angle of the image sensor, the range being between the image sensor and the toilet bowl.

11. The toilet device according to any one of claims 1 to 10,
wherein a thickness of the toilet seat is set such that buttocks and thighs of the human sitting on the toilet seat do not fall below the back surface of the toilet seat.

12. The toilet device according to any one of claims 1 to 11,
wherein an opening of the toilet seat is set to a shape such that the buttocks and the thighs of the human sitting on the toilet seat do not fall below the back surface of the toilet seat.

13. The toilet device according to any one of claims 1 to 12, further comprising an image processor configured to perform image processing on a target image acquired by the image sensor,
wherein the image processor is configured to perform the image processing of masking a region of the target image in which the human body is photobombed.

14. The toilet device according to claim 13,
wherein the image processor includes functions of detecting a dirt on a camera lens of the image sensor from the image captured by the image sensor and outputting a cleaning-requirement signal.

15. The toilet device according to any one of claims 1 to 14, further comprising a supporter configured to project from the back surface of the toilet seat to abut on an upper surface of the toilet bowl in a state in which the toilet seat is closed,
wherein the image sensor is disposed to be non-contact with the upper surface of the toilet bowl.

16. The toilet device according to any one of claims 1 to 15,
wherein the image sensor is disposed such that a center of the view field of the image sensor is directed toward a distal side from the half of the pooled water surface in the toilet bowl in a direction of viewing a pooled water surface inside the toilet bowl from the position of the image sensor.
